# EUROPÄISCHE PATENTSCHRIFT

(11) **EP 2 300 191 B1**
(45) Veröffentlichungstag und Bekanntmachung des Hinweises auf die Patenterteilung: **01.02.2012**
(21) Anmeldenummer: 09780677.2
(22) Anmeldetag: 15.07.2009
(51) Int. Cl.: B23K 26/00, G01N 33/532

(54) **VERFAHREN UND VORRICHTUNG ZUR HERSTELLUNG METALLHALTIGER ORGANISCHER VERBINDUNGEN**
METHOD AND DEVICE FOR PRODUCING ORGANIC COMPOUNDS CONTAINING METAL
PROCÉDÉ ET DISPOSITIF SERVANT À PRÉPARER DES COMPOSÉS ORGANIQUES CONTENANT DES MÉTAUX

(30) Priorität: 15.07.2008 DE 102008033070; 17.07.2008 DE 102008033570
(43) Veröffentlichungstag der Anmeldung: 30.03.2011
(73) Patentinhaber: Laser Zentrum Hannover E.V., 30419 Hannover (DE)
(72) Erfinder: BARCIKOWSKI, Stephan, 30419 Hannover (DE); PETERSEN, Svea, 18057 Rostock (DE)
(74) Vertreter: Taruttis, Stefan Georg
(86) Internationale Anmeldenummer: PCT/EP2009/059116
(87) Internationale Veröffentlichungsnummer: WO 2010/007117

(56) Entgegenhaltungen:
- WO-A1-01/83155
- PETERSEN S ET AL: "In situ bioconjugation-Novel laser based approach to pure nanoparticle-conjugates" APPLIED SURFACE SCIENCE ELSEVIER SCIENCE B.V. NETHERLANDS, Bd. 255, Nr. 10, 1. März 2009 (2009-03-01) , Seiten 5435-5438, XP002553989 ISSN: 0169-4332
- PETERSEN S ET AL: "In situ bioconjugation: single step approach to tailored nanoparticle-bioconjugates by ultrashort pulsed laser ablation" ADVANCED FUNCTIONAL MATERIALS WILEY-VCH VERLAG GMBH GERMANY, Bd. 19, Nr. 8, 23. April 2009 (2009-04-23) , Seiten 1167-1172, XP002553990 ISSN: 1616-301X
- AMENDOLA V ET AL: "Controlled size manipulation of free gold nanoparticles by laser irradiation and their facile bioconjugation" JOURNAL OF MATERIALS CHEMISTRY ROYAL SOCIETY OF CHEMISTRY UK, Bd. 17, Nr. 44, 28. November 2007 (2007-11-28), Seiten 4705-4710, XP002553991 ISSN: 0959-9428
- SLOCIK J M ET AL: "Synthesis of gold nanoparticles using multifunctional peptides" SMALL WILEY-VCH GERMANY, Bd. 1, Nr. 11, November 2005 (2005-11), Seiten 1048-1052, XP002553992 ISSN: 1613-6810

### SEQUENCE LISTING

<110> Laserzentrum Hannover e.V.
<120> Verfahren und Vorrichtung zur Herstellung metallhaltiger organischer Verbindungen
<130> M1010-C-PCT
<160> 9
<170> PatentIn version 3.5
<210> 1
   <211> 39
   <212> DNA
   <213> Artificial Sequence
<220>
   <223> Seq.-ID Nr. 1: oligonukleotid mit Spezifität für das Y-chromosom
<400> 1
   tctgtgagac gacgcaccgg tcgcaggttt tgtctcaca 39
<210> 2
   <211> 17
   <212> DNA
   <213> Artificial Sequence
<220>
   <223> Seq.-ID Nr. 2: Oligonukleotid mit Spezifität für das Y-Chromosom
<400> 2
   agagactgtg gaaccgg 17
<210> 3
   <211> 18
   <212> DNA
   <213> Artificial Sequence
<220>
   <223> Seq.-ID Nr. 3: oligonukleotid mit Spezifität für das Y-Chromosom
<400> 3
   ggcgactgtg caagcaga 18
<210> 4
   <211> 18
   <212> DNA
   <213> Artificial Sequence
<220>
   <223> Seq.-ID Nr. 4: Oligonukleotid mit Spezifität für das Y-Chromosom
<400> 4
   agcacatctc ggtccctg 18
<210> 5
   <211> 56
   <212> DNA
   <213> Artificial Sequence
<220>
   <223> Seq.-ID Nr. 5: oligonukleotid
<400> 5
   gggagggcga ugcggaucag ccauguuuac gucacuccuu gucaauccuc aucggc 56
<210> 6
   <211> 19
   <212> DNA
   <213> Artificial sequence
<220>
   <223> Seq.-ID Nr. 5: siRNA
<400> 6
   accuucaggg ucagcuugc 19
<210> 7
   <211> 16
   <212> PRT
   <213> Artificial Sequence
<220>
   <223> Seq.-ID Nr. 7: organischer Bestandteil zur Penentrationsverstärkung
<400> 7
   Arg Gln Ile Lys Ile Trp Phe Gln Asn Arg Arg Met Lys Trp Lys Lys
   1               5                   10                  15
<210> 8
   <211> 14
   <212> PRT
   <213> Artificial sequence
<220>
   <223> Seq.-ID Nr. 8: organischer Bestandteil zur Penentrationsverstärkung
<400> 8
   Gly Arg Lys Lys Lys Arg Arg Gln Arg Arg Arg Pro Pro Gln
   1 5 10
<210> 9
   <211> 24
   <212> PRT
   <213> Artificial Sequence
<220>
   <223> Seq.-ID Nr. 9: organischer Bestandteil zur Penentrationsverstärkung
<400> 9
   Tyr Gly Arg Lys Lys Arg Arg Gln Arg Arg Arg Gly Tyr Gly Arg Lys
   1 5 10 15
   Lys Arg Arg Gln Arg Arg Arg Gly
   20

## Beschreibung

Die Erfindung betrifft Vorrichtungen und damit durchführbare Verfahren zur Herstellung metallhaltiger Verbindungen, die einen metallischen nanopartikulären Bestandteil und einen organischen Bestandteil aufweisen oder daraus bestehen. Der organische Bestandteil hat vorzugsweise eine Affinität zu einem Analyten, insbesondere zu eine Zellbestandteil. Alternativ kann der organische Bestandteil ein natürliches oder synthetisches organisches Molekül sein, insbesondere ein Monomer oder Polymer.

Petersen, Jakobi und Barcikowski, Applied Surface Science 5435-5438 (2009) und Petersen und Barcikowski, Advanced Functional Materials, 1167-1172 (2009) beschreiben die Herstellung von Konjugaten aus Nanopartikeln mit Oligenukleotiden durch in-situ Ablation von Goldfolie mittels ultrakurzer Laserpulse in wässriger Lösung.

### Aufgabe der Erfindung

Aufgabe der Erfindung ist die Bereitstellung eines Verfahrens und einer zur Durchführung des Verfahrens geeigneten Vorrichtung zur Herstellung metallhaltiger Konjugate.

### Allgemeine Beschreibung der Erfindung

Die Erfindung löst die Aufgabe mit dem Verfahren und der Vorrichtung, die in den Ansprüchen definiert sind. Dabei stellt die Erfindung ein Verfahren zur Herstellung, vorzugsweise zur kontinuierlichen Herstellung von Konjugaten bereit, die einen metallischen nanopartikulären Bestandteil und einen organischen Bestandteil aufweisen oder daraus bestehen. Das Verfahren nutzt die Erzeugung aktivierter bzw. reaktiver metallhaltiger Nanopartikel durch Bestrahlung eines Metallkörpers mit Laserstrahlung und vermeidet die Veränderung bzw. Schädigung durch Laserbestrahlung von organischen Bestandteilen solcher Konjugate.

Der nanopartikuläre metallische Bestandteil umfasst oder besteht bevorzugt aus plasmonresonanten Metallen, insbesondere Au, Ag, Ti und/oder Cu. Der nanopartikuläre metallische Bestandteil der mit dem erfindungsgemäßen Verfahren hergestellten Verbindung liegt vorzugsweise in metallischer Form vor, insbesondere ausgewählt aus der Gruppe, die Gold, Silber, Titan, Platin, Iridium, Tantal, Eisen, Nickel, Kobalt und Kupfer und Mischungen dieser, insbesondere Eisen-Nickel-Legierungen und Kobalt-Samarium-Legierungen, Gold-Silber-Legierungen (AuAg), Eisen-Gold-Legierungen (FeAu) und Nickel-Titan-Legierungen (NiTi) umfasst, oder als Metalloxid, insbesondere ausgewählt aus der Gruppe, die Oxide von Titan, Zink und Eisen, insbesondere ferromagnetische Metalloxide dieser umfasst. Weiter bevorzugt ist der nanopartikuläre metallische Bestandteil ein Kern-Hülle-Partikel, dessen Kern metallisch ist und dessen Hülle das Oxid des selben Metalls ist, z.B. Zn (Kern)/ZnO (Hülle). Es hat sich gezeigt, dass das erfindungsgemäße Herstellungsverfahren, insbesondere bei Verwendung eines sauerstoffhaltigen Trägerfluids, z.B. Alkohol oder Wasser, Partikel mit einem metallischen Kern und einer metalloxidischen Hülle erzeugt.

Für die Stabilität der Bindung des metallhaltigen Bestandteils mit dem organischen Bestandteil von Konjugaten ist es bevorzugt, dass einer dieser Bestandteile eine weiche Lewis-Base ist, während der andere eine weiche Lewis-Säure ist, oder einer dieser Bestandteile eine harte Lewis-Base ist, während der andere eine harte Lewis-Säure ist, z.B. Au mit einem thiolgruppenhaltigen organischen Bestandteil oder Fe mit einem amingruppenhaltigen organischen Bestandteil.

Die Bindung zwischen nanopartikulärem metallischem Bestandteil und organischem Bestandteil ist vorzugsweise eine direkte Bindung, wahlweise kann der organische Bestandteil eine sogenannte Spacergruppe aufweisen, z.B. ein C1- bis C6- Alkyl oder ein Polyglykol, insbesondere Hexaethylenglykol, wobei die Spacergruppe an den metallischen Bestandteil bindet.

Der organische Bestandteil der metallhaltigen organischen Verbindung ist eine Lewis-Base und kann eine reaktionsfähige Gruppe aufweisen, beispielsweise ausgewählt aus C-C-Doppelbindungen, insbesondere ethylenisch ungesättigten Doppelbindungen, Carboxy-, Carbonyl, Thiol-, Sulfid- und Epoxid -Gruppen, insbesondere mit terminaler Thiolgruppe wie z.B. ein Cysteinrest, ein Alkylthiolrest oder Ethylenglycolthiol, oder ein Disulfid, z.B. ein Pyridyldisulfid, ein C1- bis C12-Alkyldisulfid, ein Ethylenglycoldisulfid, oder Liponsäure.

In bevorzugter Ausführungsform weist der organische Bestandteil eine Nukleinsäuresequenz und/oder eine Aminosäuresequenz mit einer spezifischen Affinität zu einem Analyten auf, insbesondere eine spezifische Affinität zu einem intra- oder extrazellulären Zellbestandteil einer prokaryotischen oder eukaryotischen, insbesondere einer tierischen Zelle. Bevorzugt weist der organische Bestandteil eine Nukleinsäuresequenz, auch Oligonukleotid genannt, auf, die z.B. revers komplementär, d.h. hybridisierbar zu einer Zielsequenz ist, die der Analyt ist. Besonders bevorzugt ist der organische Bestandteil eine Nukleinsäuresequenz, die für einen geschlechtschromosomenspezifischen Abschnitt einer tierischen Zelle, insbesondere einer Spermienzelle spezifisch ist.

In einer weiteren Ausführungsform weist der organische Bestandteil die ein Antigen bindenden Bestandteile eines Antikörpers auf, beispielsweise eine oder mehrere Aminosäureketten, die ein Paratop eines Antikörpers bilden, insbesondere einen natürlichen oder synthetischen Antikörper oder einen Antigen - bindenden Anteil eines Antikörpers.

Entsprechend kann der organische Bestandteil ein Bindeanteil der erfindungsgemäßen Verbindung sein, z.B. ein natürlicher oder synthetischer, einkettiger oder zweikettiger Antikörper, insbesondere eine Nukleinsäuresequenz, die vorliegend in 5' nach 3' angegeben sind, beispielsweise RNA, DNA, phosphorylierte DNA (PSNA), Peptidyl-DNA, z.B. LNA (*locked nucleic acid*, verrastete Nukleinsäure) oder PNA, oder ein Rezeptor - spezifischer Ligand, z.B. für einen zellulären Rezeptor, oder eine andere Verbindung, die spezifisch mit einem oberflächengebundenen Bestandteil einer Zelle oder einem zellintemen Bestandteil in Wechselwirkung tritt, insbesondere ein Antikörper.

Eine Nukleinsäuresequenz, die organischer Bestandteil eines erfindungsgemäßen Konjugats ist, kann z.B. eine geschlechtschromosomenspezifische, eine Allel- oder SNP -spezifische Nukleinsäuresequenz aufweisen.

Bevorzugte Nukleinsäuresequenzen sind TCT GTG AGA CGA CGC ACC GGT CGC AGG TTT TGT CTC ACA (Seq.-ID Nr. 1), die für das Y-Chromosom des Rindes spezifische Sequenz AGA GAC TGT GGA ACC GG (Seq.-ID Nr. 2), GGC GAC TGT GCA AGC AGA (Seq.-ID Nr. 3) oder AGC ACA TCT CGG TCC CTG (Seq.-ID Nr. 4), oder eine Expressionskassette, die ein Markergen kodiert, z.B. ein lumineszentes Protein, insbesondere GFP, eGFP, Red, eine für einen Krankheitsmarker spezifische Sequenz, z.B. die für das Prostata-Membranantigen spezifische GGG AGG GCG AUG CGG AUC AGC CAU GUU UAC GUC ACU CCU UGU CAA UCC UCA UCG GC (Seq.-ID Nr. 5), oder eine siRNAkodierende Sequenz, z.B. die gegen GFP gerichtete siRNA ACC UUC AGG GUC AGC UUG C (Seq.-ID Nr. 6).

Für organische Bestandteile erfindungsgemäßer Konjugate, die eine spezifische Affinität zu einem Analyten aufweisen, werden die erfindungsgemäßen Konjugate auch als Nachweis-oder Detektionskonjugate bezeichnet.

Es ist bevorzugt, dass erfindungsgemäße Konjugate, insbesondere Detektionskonjugate, in denen ein metallhaltiger Nanopartikel mit einem organischen Bestandteil verbunden ist, welcher eine spezifische Affinität für einen Analyten aufweist, zusätzlich gebundene penetrationsverstärkende Verbindungen aufweisen, beispielsweise Polyargininpeptide, insbesondere bevorzugt mit einem gebundenen Myristinsäurerest und/oder einem Transfektionsagenz, beispielsweise ausgewählt aus der Gruppe, die Fugene, Lipofectamine, Oligofectamine, Optifect, DMRIE C, AntHD, Penetratin (Seq.-ID Nr. 7, RQIKIWFQNRRMKWKK) Penetratin 43-58, HIVI-Tat Protein (Seq.-ID Nr. 8, GRICKKRRQRRRPPQ), Tat-Peptid 49-59, Tat-Peptid 48-62, Tat-Peptid 2-4, Peptide, die dieSequenz Seq.-ID Nr. 9 (YGRKIGZRQRRRGYGRKKRRQRRRG) enthalten oder daraus bestehen, amphipathische Peptide (MAPs), z.B. der Aminosäuresequenz KALA oder KLAL, cis-γ-Amino-L-Prolin-haltige Peptide, VP22, LL37, TP10, MPG, Galparan, Transportan, MPG, SynB1, Fushi-tarazu, Engrailed, pVEC, plsl, Cystein, Glycin, Hoechst 33342, Polysaccharide, insbesondere Dextran, Glucosaminglykane, insbesondere Hyaluronsäure, Heparin und Chitosan, Lipide, Polyvinylpyrrolidon, Ethylenglycol und Mischungen sowie Konjugate dieser umfasst. Alternativ oder zusätzlich können die Konjugate zur Verstärkung der Penetration in Zellen als Liposomen formuliert sein, oder in Mischung mit Liposomen formuliert sein.

Weiterhin betrifft die Erfindung die Verwendung von Detektionskonjugaten und die Verwendung des Herstellungsverfahrens von Detektionskonjugaten, die einen für einen Analyten spezifischen organischen Bestandteil aufweisen, zur durchflußzytometrischen Analyse und/oder zur durchflußzytometrischen Sortierung des Analyten. Bevorzugt ist der Analyt an einen Partikel gebunden, insbesondere ist der Analyt ein Zellbestandteil. Die Sortierung kann daher auch die Sortierung von Zellen sein.

Generell betrifft die Erfindung die Verwendung von Detektionskonjugaten und die Verwendung des Herstellungsverfahrens zur Herstellung von Detektionskonjugaten für die Analyse, wahlweise gekoppelt mit dem anschließenden Schritt der Sortierung, von Analyten durch
Anregen des metallischen nanopartikulären Bestandteil des Konjugats, z.B. durch Einstrahlen von Strahlung einer Anregungswellenlänge, die zur Anregung der
Oberflächenplasmonresonanz spezifisch ist,
Detektion des von dem Konjugat abgegebenen Signals durch Messen der abgegebenen Strahlung und
Bestimmen der Verschiebung des Emissions- oder Absorptionsmaximums.
Wahlweise können Analyten bzw. Partikel, die den Analyten aufweisen, anschließend entsprechend der bestimmten Verschiebung des Emissions- oder Absorptionsmaximums in zwei oder mehr Fraktionen sortiert werden, beispielsweise in eine Fraktion mit einer Signalintensität auf einer Seite eines Schwellenwerts, für deren Analyten der organische Bestandteil des Konjugats, insbesondere die Nukleinsäuresequenz des Konjugats spezifisch, insbesondere hybridisierbar ist, und eine Fraktion, für die Signalintensitäten auf der anderen Seite des Schwellenwerts gemessen wurden, bei dem z.B. die Nukleinsäuresequenz des Konjugats nicht hybridisiert.

Allgemein kann die Anregungswellenlänge im Bereich von 350 bis 1000 nm liegen, bevorzugt im Bereich von 450 bis 800 nm, besonders bevorzugt bei 633, 488, 514 oder 543 mm.

Insbesondere betrifft die Erfindung die Verwendung von Detektionskonjugaten und die Verwendung des Herstellungsverfahrens von Detektionskonjugaten, die einen geschlechtschromosomenspezifischen Bindeanteil aufweisen, insbesondere eine geschlechtschromosomenspezifische Nukleinsäuresequenz, zur geschlechtschromosomenspezifischen Detektion von Spermatozoen, insbesondere Verwendung von Detektionskonjugaten und die Verwendung des Herstellungsverfahrens zur Herstellung von Detektionskonjugaten für die Sortierung intakter, lebensfähiger Spermatozoen eines männlichen, nicht-menschlichen Säugetiers in eine im Wesentlichen X-chromosomenhaltige Fraktion und eine im Wesentlichen Y-chromosomenhaltige Fraktion.

In dieser Ausführungsform betrifft die Erfindung auch die Verwendung von Konjugaten, die einen metallischen nanopartikulären Bestandteil und einen organischen Bestandteil aufweisen, in einem Verfahren zur Erzeugung einer Fraktion nicht-menschlicher Spermatozoen durch Sortieren mittels eines Durchflusszytometers mit den Schritten Kontaktieren intakter, lebensfähiger Spermatozoen, die von einem männlichen, nicht-menschlichen Säugetier erhalten wurden, mit den Schritten
Vereinzeln der Spermatozoen, entweder in Tropfen einer Hüllflüssigkeit, die vorzugsweise elektrisch leitfähig und isotonisch ist, oder in einem Fluidstrom, der beispielsweise in einem Durchflusszytometer erzeugt wird,
Anregen des metallischen nanopartikulären Bestandteil des Konjugats, z.B. durch Einstrahlen von Strahlung einer Anregungswellenlänge, die zur Anregung der Oberflächenplasmonresonanz spezifisch ist,
Detektion des von dem Konjugat abgegebenen Signals durch Messen der abgegebenen Strahlung,
Bestimmen der Verschiebung des Emissions- oder Absorptionsmaximums,
Sortieren der Spermatozoen entsprechend der gemessenen Signalintensität in zumindest zwei Fraktionen der Spermatozoen, zur Herstellung von zumindest zwei Fraktionen von Spermatozoen beispielsweise einer Fraktion mit einer Signalintensität auf einer Seite eines Schwellenwerts für die geschlechtschromosomenspezifische Spermatozoenfraktion, für deren Geschlechtschromosom der organische Bestandteil des Konjugats, insbesondere die Nukleinsäuresequenz des Konjugats spezifisch, insbesondere hybridisierbar war, und eine Fraktion von Spermatozoen, für die Signalintensitäten auf der anderen Seite des Schwellenwerts gemessen wurden, bei den z.B. entsprechend die Nukleksäurcsequenz des Konjugats nicht hybridisiert.

Die Anregungswellenlänge liegt im Bereich von 350 bis 1000, bevorzugt im Bereich von 520 bis 800 nm, z.B. für Goldpartikel. Generell kann die Detektion des von dem Konjugat abgegebenen Signals durch Messen von Streulicht erfolgen; die Bestimmung Emissions- oder Absorptionsmaximums kann Bestimmung der Verschiebung des Maximums der Emissionswellenlänge sein, da die emittierte Wellenlänge durch Bindung des Konjugats an einen Analyten verschoben wird.

Bei dieser Verwendung liegt ein besonderer Vorteil darin, dass die hergestellten Konjugate jeweils spezifisch für das in einem Spermatozoon enthaltene Geschlechtschromosom ein signifikant qualitativ abweichendes detektierbares Signal bei entsprechender Anregung emittieren, und das Signal abhängig von der Hybridisierung signifikant abweicht, wobei diese Abweichung hinreichend groß ist, dass sie ohne eine spezifische Orientierung der Spermatozoen gegenüber der eingestrahlten Anregungsenergie bzw. gegenüber dem Detektor zur Aufnahme des emittierten Signals gemessen werden kann.

Entsprechend ist diese Verwendung des Verfahrens zur Herstellung von Konjugaten bei der Herstellung geschlechtschromosomenspezifischer Spermatozoenfraktionen durchführbar, vorzugsweise mit der Vereinzelung der Spermatozoen während der Detektion eines vom Konjugat abgegebenen Signals und während der anschließenden Sortierung in Fraktionen auf Basis des gemessenen Detektionssignals, auch ohne Spermatozoen entlang ihrer Längsachse auszurichten, z.B. in einem Durchflusszytometer mit der Erzeugung einer kontinuierlichen Flüssigkeitsphase, oder mit der Erzeugung eines Tropfenstroms, wobei in jedem Tropfen genau ein Spermatozoon enthalten ist.

Vorzugsweise betrifft die Verwendung bei der Herstellung einer Spermafraktion die Zuordnung vereinzelter Spermatozoen im Anschluss an die Detektion des Signals des Detektionskonjugats zu einer Fraktion, beispielsweise durch Ablenkung von Tropfen oder Volumenabschnitten der Hüll- oder Trägerflüssigkeit, die ein Spermatozoon enthalten. Die Ablenkung kann z.B. durch ein vom detektierten Signal abhängig erzeugtes elektrisches Feld erfolgen. Alternativ zu dieser Sortierung in zumindest zwei Fraktionen ist es bei der erfindungsgemäßen Verwendung auch möglich, die vereinzelten Spermatozoen im Anschluss an die Detektion in Abhängigkeit von der Höhe des Detektionssignals unbeeinflusst in dem Trägermedium bzw. der Hüllflüssigkeit zu belassen, bzw. abhängig von dem Detektionssignal einen Anteil der Spermatozoen zu inaktivieren, beispielsweise durch Erwärmen mittels gezielter Laserbestrahlung der Spermatozoen, die in der vorangegangenen Detektion einen Schwellenwert des Signals über- oder unterschritten haben. Bei dieser Variante der Verwendung von Konjugaten enthält eine erzeugte Spermatozoenfraktion die nicht inaktivierten, d.h. z.B. nicht bestrahlten Spermatozoen, und die andere Fraktion inaktivierte (nicht befruchtungsfähige) Spermatozoen, wobei die Inaktivierung abhängig vom Unterschreiten oder Überschreiten eines Schwellenwerts für das gemessene Detektionssignal erfolgt. Diese Verwendung kann daher ein Verfahren betreffen, bei dem in einem Durchflusszytometer ein Laser zu inaktivierenden Bestrahlung einzelner Zellen im kontinuierlichen Flüssigkeitsstrom in Abhängigkeit von dem Signal eingerichtet ist, das vom Detektionskonjugat emittiert wird.

Eine bevorzugte, für das Y-Chromosom insbesondere des Rindes spezifische Nukleinsäuresequenz ist Seq.-ID Nr. 4 (5' AGC ACA TCT CGG TCC CTG 3'), und alternativ kann eine Nukleinsäuresequenz der Seq.-ID Nr. 4 und/oder Nr. 5 eingesetzt werden.

Alternativ zur geschlechtschromosomenspezifischen Nukleinsäuresequenz kann ein erfindungsgemäßes Konjugat z.B. eine für ein Allel oder für einen SNP (Einzelnukleotid-Polymorphismus) spezifische Nukleinsäuresequenz enthalten, um Zellen, insbesondere Spermatozoen, Allel-spezifisch bzw. SNP-spezifisch zur fraktionieren.

Es ist bevorzugt, dass die Detektionskonjugate aus kolloidalen Goldnanopartikeln mit unmittelbar daran gebundenen geschlechtschmmosomenspezifischen Nukleinsäuresequenzen bestehen, optional zusätzlich mit unmittelbar am Goldnanopartikel gebundenen penetrationsverstärkenden Verbindungen. Die Detektionskonjugate weisen daher die geschlechtschromosomenspezifische Nukleinsäuresequenz in unmittelbarer Bindung an kolloidale Metallnanopartikel, insbesondere kolloidale Goldnanopartikel auf, gegebenenfalls zusätzlich unmittelbar an den Nanopartikel gebundene penetrationsverstärkende Verbindungen.

Für die Anregung der Detektionskonjugate, die einen kolloidalen Goldnanopartikel aufweisen, kann bei der Verwendung zur Fraktionierung von Spermatozoen beispielsweise Licht mit einer Wellenlänge von 350 bis 1000 nm, bevorzugt 450 bis 1000 nm, besonders bevorzugt etwa 800 nm zur Bestrahlung verwendet werden. Als Signal für die geschlechtschromosomenspezifische Detektion kann die Absorption der Anregungsstrahlung gemessen werden, wobei vorzugsweise die geschlechtschromosomenspezifisch Hybridisierung der Nukleinsäuresequenz eines Konjugats als Veränderung der Absorption detektiert wird, gegebenenfalls durch Verschiebung der Wellenlänge, insbesondere zu höheren Wellenlängen.

Aufgrund der Detektion der vom Konjugat emittierten Strahlung bei der Verwendung zur Detektion eines Analyten, optional mit anschließender Sortierung in Abhängigkeit von dem detektierten Signal des Konjugats als Änderung der Lumineszenz, Absorption und/oder Streuung, wahlweise mit Bestimmung der Wellenlängenverschiebung, ermöglicht die Erfindung die Verwendung des Konjugats für Analyse- und Sortierverfahren für Zellen, insbesondere für Spermatozoen mit den Schritten der berührungslosen Detektion der geschlechtschromosomenspezifischen Hybridisierung und der anschließenden Fraktionierung und/oder Inaktivierung vereinzelter Spermatozoen in Abhängigkeit von dem detektierten Signal.

Das erfindungsgemäße Verfahren sieht den Abtrag eines Metallkörpers in einem Trägerfluid mittels Laserbestrahlung vor, wobei das Trägerfluid während der Laserbestrahlung des Metallkörpers über dessen Oberfläche bewegt, z.B. gepumpt wird. Durch die Laserbestrahlung erzeugte metallhaltige Nanopartikel werden durch die erzwungene Bewegung des Trägerfluids aus dem Bereich des Laserstrahls bewegt, so dass metallhaltige Nanopartikel nach Kontaktierung bzw. Reaktion mit einer Vorläuferverbindung des organischen Bestandteils im wesentlichen nicht durch den Laserstrahl treten. Für die Zwecke der Erfindung umfasst der Begriff des Metallkörpers neben einstückigem Metall oder Metalloxid auch partikuläres Metall oder Metalloxid, insbesondere Metallpulver oder Metalloxidpulver, das optional verfestigt ist und/oder ein Bindemittel enthalten kann.

Das beim Herstellungsverfahren eingesetzte Trägerfluid ist vorzugsweise eine Flüssigkeit, z.B. ausgewählt aus der Gruppe, die aus wässrigen Zusammensetzungen, insbesondere reinem, salzfreien Wasser, wässrigem Puffer mit Tris, HEPES, MES, Imidazol, Glycin und/oder Triethanolamin, Wasser oder solcher wässriger Puffer mit einem organischen Lösungsmittel, z.B. ausgewählt aus der Gruppe, die C1- bis C5-Alkohol, insbesondere Ethanol, Propanol, Butanol, Aceton, Formaldehyd sowie THF und Mischungen von zumindest zweien dieser umfasst. Alternativ kann das Trägerfluid aus der Gruppe der vorgenannten organischen Lösungsmittel und Mischungen dieser ausgewählt sein, vorzugsweise ist das Trägerfluid THF oder Aceton.

Dem Trägerfluid wird zeitlich vor oder nach Einwirkung der Laserstrahlung auf den Metallkörper eine Vorläuferverbindung eines organischen Bestandteils des Konjugats zugesetzt. Die in dem Trägerfluid enthaltene Vorläuferverbindung bildet mit den mittels Laserbestrahlung des Metallkörpers erzeugten metallhaltigen Nanopartikeln ohne weiteres Bindungen aus, so dass die Konjugate z.B. auch ohne reaktive bifunktionale Kopplungsreagenzien erzeugt werden. Vorzugsweise haben die Vorläuferverbindungen zumindest eine mit den metallhaltigen Nanopartikeln innerhalb kurzer Zeit (insbesondere innerhalb 0,5 µs bis 100 ms) nach deren Erzeugung mittels Laserbestrahlung reaktive Gruppe.

In einer alternativen Ausführungsform ist die Laserstrahlung Ultrakurzpulslaserstrahlung. Es hat sich gezeigt, dass die Veränderung von Konjugaten durch die Laserstrahlung durch Ultrakurzpulslaserstrahlung vermieden wird, während ausreichend Nanopartikel durch Bestrahlung eines Metallkörpers erzeugt werden, die mit organischer Vorläuferverbindung im Trägerfluid zu einem Konjugat reagieren. Gegenwärtig wird die Vermeidung von Veränderungen erzeugter Konjugate bei einem Herstellungsverfahren mit Ultrakurzpulslaserstrahlung auf eine Verfahrensführung zurückgeführt, bei der die Pulsdauer, die z.B. kürzer als 1 bis 100 ps ist, kürzer als die Relaxationszeit des metallhaltigen Nanopartikels ist. Entsprechend kann diese Ausführungsform ohne die Erzeugung einer Bewegung des Trägerfluids über den Metallkörper sein.

In dieser Ausführungsform werden die Detektionskonjugate, die vorzugsweise kolloidale Goldnanopartikel sind, die mit einem geschlechtschromosomenspezifischen Oligonukleotid, insbesondere PNA konjugiert sind, dadurch hergestellt, dass Nanopartikel mittels eines Ultrakurzpulslasers von Gold in wässrigem Medium als Trägerfluid abgetragen werden, und in dem wässrigen Medium die Nukleinsäuresequenz, optional zusätzlich gleichzeitig oder später zugegeben penetrationsverstärkendes Agenz vorliegt. Die Erzeugung der Goldnanopartikel durch Laserabtragung mittels Ultrakurzpulsen stellt Nanopartikel mit reaktiver Oberfläche her, die auch partielle oxidierte Au+, Au3+ an der Oberfläche aufweisen können. Überraschenderweise ist gefunden worden, dass allein die Erzeugung von Metallnanopartikeln durch Ultrakurzpuls-Laserabtragung in Anwesenheit der geschlechtschromosomenspezifischen Nukleinsäuresequenz, gegebenenfalls auch für in Mischung oder später zugegebene penetrationsverstärkende Agenzien eine unmittelbare Bindung der Nukleinsäuresequenz bzw. des penetrationsverstärkenden Agenzes mit dem Goldnanopartikel ergibt. Durch die Ultrakurzpuls-Laserabtragung werden die Metallpartikel, insbesondere Goldnanopartikel, partiell oxidiert und wirken als Elektronenakzeptoren, die mit Bindeanteilen, insbesondere Nukleinsäuresequenzen, und mit wahlweise gleichzeitig oder später anwesenden penetrationsverstärkenden Agenzien eine Bindung eingehen, z.B. eine Komplex- oder koordinative Bindung, so dass im Konjugat das Metall in metallischer Form, insbesondere nicht als Metalloxid vorliegt.

Zur Erhöhung der Bindungsstärke können Nukleinsäuresequenzen, bzw. das penetrationsverstärkende Agenz (die penetrationsverstärkende Verbindung) mit mit Gold reaktiven Gruppen versehen sein, insbesondere mit Thiol-, Carboxy- Amid- und/oder Amingruppen, die Nukleinsäuresequenzen am 3'- und/oder 5'-Ende, vorzugsweise am 3' Ende zur Bindung an einen Nanopartikel. Es ist möglich, dieses Herstellungsverfahren kontinuierlich in einer Durchflusskammer durchzuführen, wobei wässriges Medium mit einem Gehalt an Nukleinsäuresequenz am Gold wobeiströmen gelassen wird, während von dem Gold kolloidale Nanopartikel durch Einstrahlung von Ultrakurzpulslaserstrahlung hergestellt werden. In dieser Ausführungsform können penetrationsverstärkende Agenzien in einem gewünschten Verhältnis in Mischung mit Nukleinsäuresequenzen eingesetzt werden, oder penetrationsverstärkende Agenzien können stromabwärts des Ortes der Erzeugung der kolloidalen Goldnanopartikel dem Fluidstrom zugegeben werden, sodass nach Reaktion der Nanopartikel mit Nukleinsäuresequenzen reaktive Stellen der Nanopartikel mit penetrationsverstärkendem Agenz reagieren können.

Erfindungsgemäß werden magnetische Nanopartikel erzeugt, z.B. Nanopartikeln mit einem Gehalt an oder bestehend aus Fe, Fe-oxid und/oder einer Fe-Legierung, die dann durch Detektion der Verschiebung der Relaxation bei Kopplung an das spezifische Geschlechtschromosom detektierbar sind, z.B. durch Detektion des Relaxationsunterschieds aufgrund der spezifischen Bindung des Detektionskonjugats an oder in Säugerspermatozoen, z.B. des Relaxationsunterschieds zwischen X-chromosomhaltigen Spermatozoen gegenüber Y-chromosomhaltigen Spermatozoen bei geschlechtschromosomenspezifischer Nukleinsäuresequenz zur anschließenden Selektion der Spermatozoen. Bei unspezifischer Nukleinsäuresequenz des Detektionskonjugats kann die Detektion und Selektion auf Basis des quantitativen Relaxationsunterschieds erfolgen, da sich Spermatozoen auch durch den unterschiedlichen Gesamt-DNA-Gehalt unterscheiden.

Alternativ zur geschlechtschromosomenspezifischen Nukleinsäuresequenz kann daher eine zufällige bzw. willkürliche Nukleinsäuresequenz und/oder ein in DNA interkalierender Stoff, z.B. ein Farbstoff, insbesondere Höchst Bisbenzimind 33342, als organischer Bestandteil in den Detektionskonjugaten enthalten sein, sodass zur Identifikation anhand des Geschlechtschromosoms ein quantitativer Unterschied des Signals wegen des geringeren Gesamt-DNA-Gehalts der Y-chromosomenhaltigen Spermatozoen zu detektieren ist.

Die Nanopartikel, die in erfindungsgemäßen Detektionskonjugaten enthalten sind, sind vorzugsweise hergestellt durch Ultrakurzpuls-Laserabtragung eines Metalls im wässrigen Medium, z.B. eingetaucht in eine wässrige Zusammensetzung, wobei der Ultrakurzpuls eine Pulsdauer von 10 fs bis 15 ps hat, bei einer Wellenlänge von größer 330 nm und maximal 1030 nm, insbesondere im Bereich von 500 bis 1000 nm. Die Zeitdauer der Abtragung beträgt vorzugsweise ca. 10 bis 200 s, z.B. 40 bis 60 s, insbesondere 53 s, bei einer Pulsenergie von ca. 50 bis 200 µJ, insbesondere 80 bis 120 µJ, vorzugsweise 120 µJ, die Pulsdauer ca. 100 bis 140 fs, insbesondere ca. 120 fs, vorzugsweise bei 800 nm.

Das erfindungsgemäße Herstellungsverfahren ergibt Nanopartikel, die auch mit gebundenem Bindeanteil, der z.B. ein Peptid oder eine geschlechtschromosomenspezifische Nukleinsäuresequenz, vorzugsweise als PNA ist, eine für die Penetration der Zellwand von Säugerspermatozoen, insbesondere des Rindes besonders geeignete Größe und/oder Konformation haben. Die Nanopartikel haben z.B. eine Größe von 1 bis 150 mm, bis 100 nm, vorzugsweise 5 bis 50 nm oder bis 25 nm.

Das Herstellungsverfahren für Detektionskonjugate mit einem Gehalt an Nanopartikeln erzeugt in dieser Ausführungsform wegen der Erzeugung von Nanopartikeln mittels Ultrakurzpulslaserstrahlung bei geringer Wärmeeinwirkung auf Bestandteile der Konjugate oder auf das Trägerfluid Detektionskonjugate in einer sehr geringen Zeitspanne, z.B. von 1 bis 10 ps, innerhalb welcher eine hohe Reaktivität der metallischen Nanopartikel vorliegt. Die Nanopartikel weisen diese Reaktivität unmittelbar nach ihrer Erzeugung beispielsweise mit Thiol-haltigen Nukleinsäuresequenzen auf, während im Anschluss an diese Zeitspanne die Agglomeration der Nanopartikel einsetzt. Die geringe Wärmeeinwirkung ist vorteilhaft, da dadurch eine Schädigung der organischen Bestandteile reduziert wird, und vorzugsweise im Wesentlichen vermieden wird. Entsprechend weisen die für das Herstellungsverfahren einzusetzenden Nukleinsäuresequenzen vorzugsweise Thiol-, Keto-, Carboxy-, Amid- oder Amingruppen oder Phosphingruppen auf, um eine entsprechende koordinative Bindung zum Goldnanopartikel herzustellen, d.h. ohne die Verwendung eines zusätzlichen Kopplungsreagenzes zwischen Nukleinsäuresequenz und Nanopartikel, sodass z.B. das Detektionskonjugat aus metallischen Nanopartikel und Nukleinsäuresequenz besteht, welche eine reaktive Gruppe, insbesondere endständig, aufweist, beispielsweise eine Thiol-, Keto-, Carboxy-, Amid oder Amingruppe, oder Phosphingruppe, die mit dem Nanopartikel eine koordinative Bindung eingegangen ist.

In bevorzugter Ausführungsform sieht das Verfahren zur Herstellung von Konjugaten mit metallischem nanopartikulärem Bestandteil und organischem Bestandteil vor, ein Trägerfluid, das insbesondere eine Trägerflüssigkeit ist, mittels einer Zirkulations- oder Pumpeinrichtung über die Oberfläche eines Metalls zu bewegen und das Metall mit einem Laser zu bestrahlen. Durch die Laserbestrahlung des Metalls werden metallische Nanopartikel erzeugt. Das Trägerfluid kann eine Vorläuferverbindung des organischen Bestandteils des Konjugats aufweisen, oder diese Vorläuferverbindung kann dem Trägerfluid stromabwärts des Metalls zugesetzt werden.

Durch die Bewegung des Trägerfluids über die Oberfläche des Metalls während der Bestrahlung mit Laserstrahlung werden erzeugten Nanopartikel aus dem unmittelbaren Bereich des Laserstrahls bewegt und abhängig von der Repetitionsrate des Lasers in reduziertem Ausmaß bzw. nicht mehr vom Laserstrahl angeregt. Bevorzugt hat daher hat auch dieser Ausführungsform die Laserstrahlung, abhängig von ihrer Repititonsrate, im Wesentlichen eine reduziert bzw. keine Wirkung auf organische Bestandteile bereits gebildeter Konjugate, die einen an den metallischen Bestandteil gebunden organischen Bestandteil aufweisen. Dies hat den Vorteil, dass im Trägerfluid enthaltene Vorläuferverbindung mit den reaktiven Nanopartikeln unmittelbar im Anschluss an ihre Erzeugung durch Laserbestrahlung reagieren können, jedoch aufgrund der Bewegung des Trägerfluids außerhalb des Volumenabschnitts des Trägerfluids, das vom Laserstrahl durchquert wird.

Es hat sich gezeigt, dass auch bei der Erzeugung von Nanopartikeln mittels kontinuierlicher Laserbestrahlung aus einer metallischen Oberfläche in einem Trägerfluid, das Vorläuferverbindung enthielt, bei Bewegung des Trägerfluids über die Oberfläche des Metalls die erzeugten Konjugate in höherer Ausbeute als mit einem Ultrakurzpulslaser hergestellt wurden und/oder keine Veränderungen der Vorläuferverbindung aufwiesen, ausgenommen die reaktiven Gruppen der Vorläuferverbindung, die eine Bindung mit dem Nanopartikel eingehen, beispielsweise Thiol-, Keto-, Carboxy- und Amid-Gruppen. So konnte z.B. für Oligonukleotide gezeigt werden, dass bei der erfindungsgemäßen Verfahrensführung mit Bewegung des Trägerfluids über die Oberfläche des Metalls während der Laserbestrahlung, wenn das Trägerfluid Vorläuferverbindung bereits stromaufwärts der Oberfläche des Metalls enthielt, im Wesentlichen keine Veränderungen des Oligonukleotids auftraten, ausgenommen die Bindung an den Nanopartikel. Im Unterschied dazu konnten bei Erzeugung von Nanopartikeln mittels hoher Laserenergie bei statischer Verfahrensführung, d.h. ohne Bewegung des Trägerfluids, chemische Veränderungen des organischen Bestandteils von Konjugaten nachgewiesen werden, und es wird gegenwärtig angenommen, dass diese Änderungen auf die Laserbestrahlung von Konjugaten zurückzuführen ist.

Es ist bevorzugt, dass das Trägerfluid durch das Pumpen des Trägerfluids in dem Behälter bewegt wird, in dem das Metall angeordnet ist und von einem kontinuierlich strahlenden Laser bestrahlt werden kann. In einer einfachen Ausführungsform kann das Pumpen durch einen Rührer als Pumpeinrichtung innerhalb des Behälters erzeugt werden, wobei der Behälter satzweise mit Trägerfluid, das Vorläuferverbindung enthält, beschickt wird, und unter Rühren ein innerhalb des Trägerfluids angeordnetes Metall oder Metalloxid mit kontinuierlicher Laserstrahlung bestrahlt wird. Alternativ kann die Zirkulationseinrichtung eine an den Behälter angeschlossene Zirkulationseinrichtung sein, die eine Pumpeinrichtung zur Bewegung des Trägerfluids aufweist. Vorzugsweise weist der Behälter einen Zulauf für Vorläuferverbindung und/oder eine Auslassöffnung auf.

Bei der Beschreibung umfasst der Begriff Vorläuferverbindung sowohl Vorläuferverbindungen und Vorläufersubstanzen für den organischen Bestandteile eines erfindungsgemäßen Konjugats, insbesondere einen für einen Analyten spezifischen organischen Bestandteil, als auch unspezifische organische Vorläuferverbindungen und - substanzen, wie beispielsweise Vorläuferverbindungen von penetrationsverstärkenden oder unspezifischen organischen Verbindungen.

Bei der Beschreibung des erfindungsgemäßen Verfahren zur Herstellung von Konjugaten gelten Beschreibungen von Verfahrensschritten auch als die entsprechende Einrichtung von Elementen einer Vorrichtung, die für das Verfahren geeignet ist, für die jeweils beschriebenen Verfahrensschritte.

In einer bevorzugten Ausführungsform wird das Trägerfluid mittels einer Pumpe oder von einer druckbelasteten Flüssigkeitsquelle durch eine erste Einlassöffnung in eine Durchflusszelle gepumpt, in welcher das Metall fixiert ist und von einem Laserstrahl bestrahlt wird, und tritt anschließend durch eine Auslassöffnung aus der Durchflusszelle aus.

Besonders bevorzugt bildet das Metall einen Abschnitt der inneren Oberfläche der Durchflusszelle und ist beispielsweise auf einer inneren Oberfläche der Durchflusszelle angeordnet. Die Durchflusszelle weist bevorzugt einen Durchtrittsquerschnitt angrenzend an die Oberfläche des Metalls von maximal 7 cm², bevorzugt maximal 2 cm², bevorzugter maximal 1 cm², bevorzugter maximal 1 bis 50 mm² oder bis 20 mm² auf, sodass Trägerfluid nur in einem begrenzten Abstand zur Metalloberfläche an diesem vorbei bewegt wird, und in dem Trägerfluid enthaltene Vorläuferverbindung oder dem Trägerfluid im Anschluss an die Laserbestrahlung des Metalls, d.h. stromabwärts des Laserstrahls bzw. des Metalls dem Trägerfluid zugeführte Vorläuferverbindung nur in einem begrenzten Volumenabschnitt angrenzend an die Metalloberfläche vorhanden ist, und dort mit den erzeugten Nanopartikeln reagiert, während in einem großen Abstand zur Metalloberfläche, in dem unmittelbar nach Laserbestrahlung der Metalloberfläche keine Nanopartikel vorhanden sind, im Wesentlichen kein Trägerfluid und keine Vorläuferverbindung vorhanden sind.

Der Laserstrahl, der auf die Oberfläche des Metalls im Behälter bzw. Durchflusskanals gerichtet ist, wird vorzugsweise von einem Laser erzeugt und tritt durch einen für die Laserstrahlung transparenten Wandabschnitt des Behälters bzw. der Durchflusskammer ein. Vorzugsweise hat die Durchflusskammer eine Höhe oberhalb des Metallkörpers von 100 µm bis 4 mm, so dass Trägerfluid in einer Schichtdicke von 100 µm bis 4 mm über den Metallkörper strömen kann. Vorzugsweise ist die Kammer im Wesentlichen vertikal angeordnet, oder zur Durchströmung in vertikaler Richtung ausgerichtet, wobei der Laserstrahl auf eine Oberfläche des Metallkörpers gerichtet ist, die im Wesentlichen vertikal angeordnet ist. Der Laserstrahl kann etwa horizontal auf die Oberfläche des Metallkörpers ausgerichtet sein.

Aufgrund der erfindungsgemäßen Bewegung des Trägerfluids über die Oberfläche des Metalls während der Laserbestrahlung des Metalls werden durch die Laserbestrahlung erzeugte Nanopartikel aus dem vom Laserstrahl durchquerten Volumenabschnitt des Behälters bzw. des Durchflusskanals bewegt. Als Folge ist es möglich, dass die Laserstrahlung Ultrakurzpulslaserstrahlung ist, oder kontinuierliche Laserstrahlung, die beispielsweise von einem CW-Laser, Festkörperlaser, z.B. einem ND-YAG Laser, Erbium-YAG-Laser, Ti-Saphir-Laser oder einem Faser bzw. Diodenlaser erzeugt wird.

Es ist bevorzugt, dass die auf die Metall- oder Metalloxidoberfläche auftreffende Laserstrahlung relativ zum Metallkörper bewegt wird. Die Relativbewegung von Laserstrahlung zum Metallkörper kann durch eine spiralförmige oder mäandernde Führung des Laserstrahls über die Metall- oder Metalloxidoberfläche erfolgen, oder durch eine derartige Bewegung der Kammer bei feststehender Ausrichtung der Laserstrahlung.

Zur Erzeugung einer Relativbewegung von Laserstrahl zur Oberfläche des Metalls kann der Metallkörper, oder die die Durchflusskammer, auf einer beweglichen Fixiervorrichtung fixiert sein, und/oder der Laserstrahl kann über die Oberfläche des Metalls bewegt werden, beispielsweise durch Bewegung eines Umlenkspiegels, der den Laserstrahl vom Lasermedium auf die Oberfläche des Metalls richtet.

Das Trägerfluid kann gekühlt werden, vorzugsweise auf eine Temperatur oberhalb seines Erstarrungspunkts, z.B. auf maximal 20°C, bevorzugt auf 1 bis 10 oder bis 5°C, Trägerfluid mit oder aus organischem Lösungsmittel auf unterhalb 0°C. Entsprechend weist die erfindungsgemäße Vorrichtung bevorzugt eine Kühleinrichtung zur Kühlung des Trägerfluids, auf diese Temperaturen auf.

Bevorzugt werden während des Verfahrens stromabwärts des Metallkörpers weitere Vorläuferverbindungen zugesetzt, beispielsweise solche von penetrationsverstärkenden Agenzien.

In einer weiteren bevorzugten Ausführungsform, in der Trägerfluid kontinuierlich, weniger bevorzugt stoßweise, über die Oberfläche des Metalls bewegt wird, wird ein Volumenabschnitt des Durchflusskanals stromabwärts des Metalls und/oder stromabwärts des Laserstrahls von einem Sensor erfasst. Vorzugsweise ist der Sensor ein Spektrometer, insbesondere ein Spektrophotometer. Alternativ oder zusätzlich kann ein Volumenabschnitt stromaufwärts des Metalls und/oder stromaufwärts des Laserstrahls von einem Sensor gemessen werden, insbesondere von einem Spektrometer. Die von zumindest einem Spektrometer aufgenommenen Messwerte können wahlweise zur Steuerung der Positionierung des Laserstrahls, zur Steuerung Positionierung des Metalls und/oder zur Steuerung der Laserbestrahlung nach Intensität oder Bewegung relativ zur Oberfläche des Metallkörpers, insbesondere bei Ultrakurzpulslaserstrahlung zur Steuerung der Frequenz und/oder Pulsdauer eingesetzt werden, z.B. durch Kopplung der von einem Spektrometer aufgenommenen Messwerte mit einer Steuerungseinheit, die die Bewegung der Fixiereinrichtung für das Metall, die Positionierung des Laserstrahls und/oder die Erzeugung des Laserstrahls und/oder die Zudosierung von Vorläuferverbindung kontrolliert.

Vorzugsweise enthält das Verfahren im Anschluss an die Erzeugung von Konjugaten den Schritt der Abtrennung zumindest eines Teils des Trägerfluids und/oder den Schritt der Abtrennung nicht zu Konjugat umgesetzer Vorläuferverbindungen oder nicht umgesetzter Nanopartikel von den erzeugten Konjugaten, z.B. nach Austritt von Trägerfluid mit darin enthaltenen Konjugaten durch die Auslassöffnung 3. Zur Abtrennung nicht umgesetzter Nanopartikel oder Vorläuferverbindung vom Konjugat können herkömmliche Trennverfahren eingesetzt werden, insbesondere chromatographische Verfahren, beispielsweise eine Größenausschlußchromatographie oder eine affinitätschromatographische Abtrennung von Konjugaten mit einem Chromatographiemedium, das immobilisierten Analyten aufweist, insbesondere wenn die erste Vorläuferverbindung, bzw. der daraus hergestellte organische Bestandteil des Konjugats eine spezifische Affinität zum Analyten aufweist. Für die Abtrennung kann wahlweise ein zweiter chromatographischer Schritt durchgeführt werden, der für den metallischen Bestandteil von Konjugaten spezifisch ist, beispielsweise im Falle ferromagnetischer Nanopartikel, die Abtrennung von Konjugat von Trägerfluid mittels eines magnetischen Feldes, mittels Zentrifugation und/oder mittels AFFFF (asymmetrische Fluss-Feld-Fluss-Fraktionierung).

### Genaue Beschreibung der Erfindung

Die Erfindung wird nun genauer mit Bezug auf die Figuren anhand von Beispielen beschrieben, in denen
- Figur 1 eine schematische Ansicht einer einfachen Vorrichtung zur Durchführung des erfindungsgemäßen Verfahren zeigt,
- Figur 2 eine bevorzugte Ausführungsform einer Vorrichtung zur Durchführung des kontinuierlichen Verfahrens zeigt,
- Figur 3 schematisch eine weitere Ausführungsform einer Vorrichtung zur Durchführung des erfindungsgemäßen Verfahren zeigt.

In den Figuren bezeichnen gleiche Bezugsziffern funktionsgleiche Elemente.

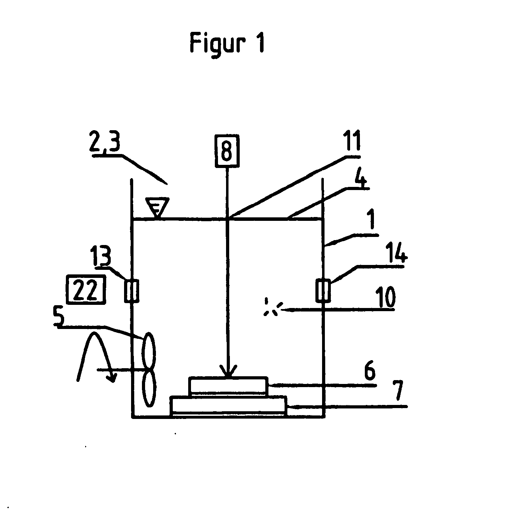

Eine einfache, zur Verwendung für das Verfahren geeignete Vorrichtung ist in Figur 1 gezeigt, die einen Behälter 1 mit einer ersten Einlassöffnung 2 und einer Auslassöffnung 3, hier in Form einer gemeinsamen Öffnung aufweist. Zur Erzeugung einer Bewegung des Trägerfluids 4 ist eine Pumpeinrichtung 5 in Kontakt mit dem Trägerfluid 4 angeordnet, beispielsweise innerhalb des Behälters 1. Wie schematisch gezeigt, kann die Pumpeinrichtung 5 in dieser Ausführungsform ein Rührer sein.

Das Metall wird in Form eines Metallkörpers 6, der einstückiges oder partikuläres Metall und/oder Metalloxid aufweist oder daraus besteht, mittels einer Fixiereinrichtung 7 in dem Behälter 1 fixiert. Die Fixiereinrichtung 7 kann auch ein einseitig offener Behälter sein, wenn der Metallkörper 6 pulverförmig ist. Ein Laser 8, der ein Lasermedium in Verbindung mit optischen Elementen zur Erzeugung eines Laserstrahls aufweist, ist so angeordnet, dass der Laserstrahl gegen den Abschnitt der Fixiereinrichtung 7 gerichtet ist, auf welchem der Metallkörper 6 anzuordnen ist. Bevorzugt wird der vom Laser 8 erzeugte Laserstrahl mittels eines Spiegels 9 gesteuert, wobei der Spiegel 9 beweglich und gesteuert ist, um eine Bewegung des Laserstrahls gegenüber dem Abschnitt der Fixiereinrichtung 7 zu erlauben, in welchem der Metallkörper 6 angeordnet ist.

Alternativ oder zusätzlich kann die Fixiereinrichtung 7 mit einer Steuerungseinheit verbunden und gesteuert beweglich sein, um ihren Abschnitt, in dem der Metallkörper 6 anzuordnen ist, gegenüber dem Laserstrahl zu bewegen.

Mittels der ersten Einlassöffnung 2 kann dem Trägerfluid 4 eine erste Vorläuferverbindung 10 zugesetzt werden, die einen organischen Bestandteil des Konjugats mit dem metallischen Nanopartikel bildet, welcher von dem Laserstrahl aus dem Metallkörper 6 erzeugt wird.

Optional kann eine zweite und weitere Vorläuferverbindung zugesetzt werden, beispielsweise penetrationsverstärkende Agenzien, die wie die erste Vorläuferverbindung 10 mit dem metallischen Nanopartikel unmittelbar nach seiner Erzeugung durch die Laserbestrahlung zu einem Konjugat reagieren.

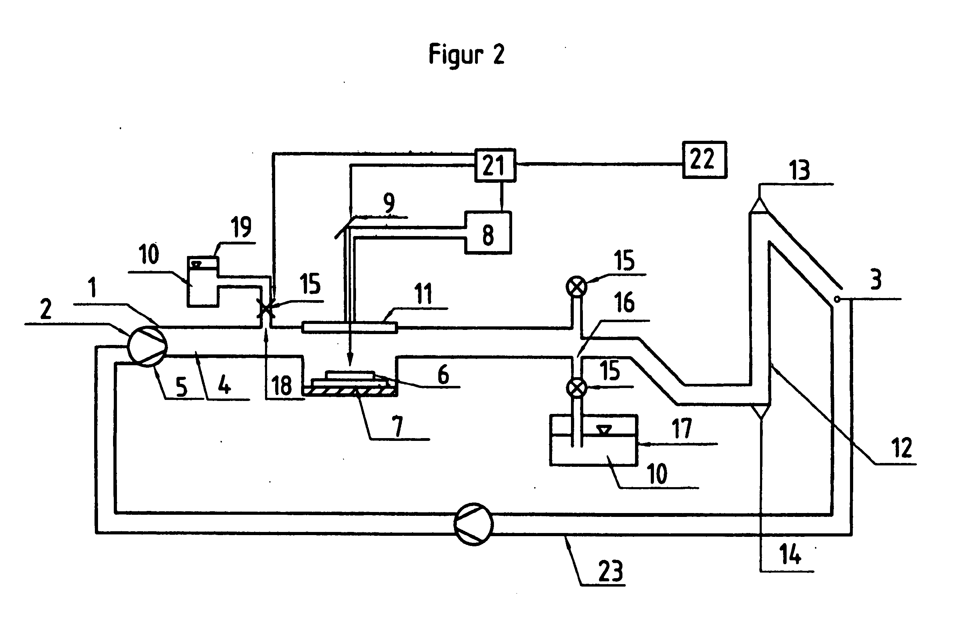

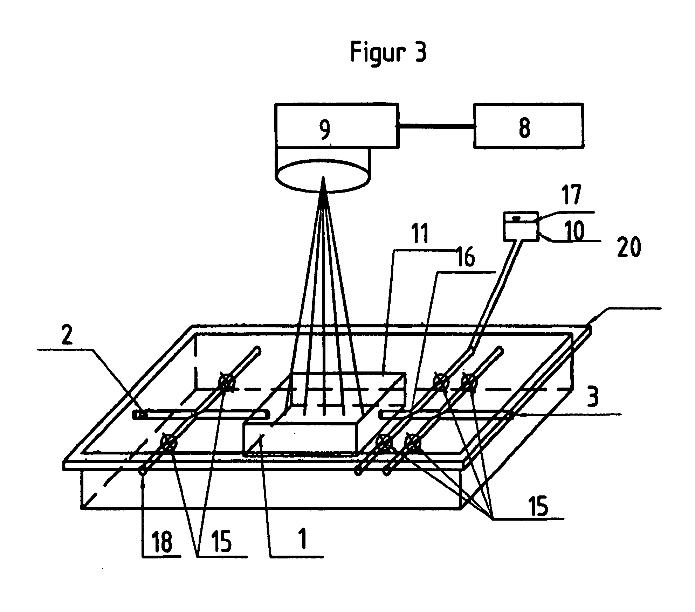

Die Figuren 2 und 3 zeigen Ausführungsformen, in denen der Behälter 1 als Strömungskanal ausgeführt ist, der beim Verfahren kontinuierlich oder stoßweise mit Trägerfluid durchströmt wird. Entsprechend können die mit Bezug auf diese Figuren genannten Eigenschaften von Vorrichtung oder Verfahren jeweils miteinander und/oder mit den in der voranstehenden Beschreibung genannten Eigenschaften kombiniert werden.

Figur 2 zeigt eine bevorzugte Ausführungsform einer Vorrichtung zur Verwendung bei der Herstellung erfindungsgemäßer Vorrichtungen zur Verwendung in einem Verfahren zur Herstellung von Konjugaten, bei der der Behälter 1 als Strömungskanal ausgebildet ist. Der Behälter 1 weist an seinem ersten Ende eine erste Einlassöffnung 2 auf und an seinem gegenüberliegenden zweiten Ende eine Auslassöffnung 3. In einem Abschnitt des als Strömungskanal ausgebildeten Behälters 1 ist eine Fixiereinrichtung 7 zur Aufnahme eines Metallkörpers 6 angeordnet. Vorzugsweise ist der als Strömungskanal ausgebildete Behälter 1 umfänglich geschlossen und weist gegenüber der Fixiereinrichtung 7 einen für die verwendete Laserstrahlung transparenten Abschnitt 11 auf. Entsprechend ist der Laser 8, wahlweise mit einem Spiegel 9 zur gesteuerten Umlenkung, so angeordnet, dass der Laserstrahl durch den transparenten Abschnitt des als Strömungskanal ausgebildeten Behälters 1 auf den Abschnitt der Fixiereinrichtung 7 gerichtet ist, in welchem der Metallkörper 6 anzuordnen ist.
Generell kann die erste Vorläuferverbindung 10 dem Trägerfluid vor oder nach Eintritt in die erste Einlassöffnung zugegeben werden. Vorzugsweise weist der als Strömungskanal ausgebildete Behälter 1 unmittelbar stromabwärts der Fixiereinrichtung 7 eine zweite Einlassöffnung 16 auf, an die ein zweiter Vorratsbehälter 19 für eine erste Vorläuferverbindung 10 und/oder für eine zweite Vorläuferverbindung, z.B. eine Vorläuferverbindung eines penetrationsverstärkenden Agenzes, angeschlossen ist. Die Verbindungsleitung zur zweiten Einlassöffnung 16 weist vorzugsweise eine Dosiereinrichtung 15 auf, die für die Zwecke der Beschreibung auch als Dosierventil 15 bezeichnet wird.

Der als Strömungskanal ausgebildete Behälter 1 kann zusätzlich oder alternativ zu zweiten Einlassöffnungen 16 eine oder mehr dritte Einlassöfinungen 18 auf, die in einem Abschnitt des Strömungskanals zwischen der ersten Einlassöffnung 2 und der Fixiereinrichtung 7 angeordnet ist. Die dritte Einlassöffnung 18 ist mit einem zweiten Vorratsbehälter 19 gekoppelt und über ein weiteres Dosierventil 15, das in der Verbindungsleitung zwischen zweitem Vorratsbehälter 19 und dritter Einlassöffnung 18 angeordnet ist, steuerbar. Der zweite Vorratsbehälter 19 kann z.B. mit einer ersten 10 und/oder zweiten Vorläuferverbindung gefüllt sein.

Vorzugsweise weisen daher die Leitungen, die die erste Einlassöffnung 2 mit dem ersten Vorratsbehälter für Trägerfluid, eine zweite Einlassöffnung 16 und eine dritte Einlassöffnung 18 mit dem jeweils zugeordneten zweiten bzw. dritten Vorratsbehälter verbinden, jeweils steuerbare Dosierventile 15 auf, die besonders bevorzugt mit einer Steuereinheit 21 verbunden sind.

Für die Kontrolle und/oder Steuerung der Vorrichtung und des Verfahrens weist die Vorrichtung einen Sensor 22 auf, der eine Eigenschaft der Inhaltsstoffe des Trägerfluids detektiert. Vorzugsweise ist der Sensor 22 ein Spektrometer, insbesondere ein Spektrophotometer, dessen Detektionsbereich zumindest ein Abschnitt des Innenvolumens des Behälters 1 ist. Besonders bevorzugt weist der als Strömungskanal ausgebildete Behälter 1 in einem Abschnitt zwischen der Fixiereinrichtung 7 und der Auslassöffnung 3, d.h. stromabwärts der Fixiereinrichtung 7, bzw. stromabwärts des Laserstrahls, in welchem dieser durch das Innenvolumen des Behälters 1 tritt, einen Küvettenabschnitt 12 auf, der einen gegenüber dem Durchmesser des Strömungskanals größere Distanz zwischen zwei beabstandeten Küvettenwänden 13, 14 aufweist, wobei Detektor an einer ersten Küvettenwand 13 und/oder einer zweiten Küvettenwand 14 angeordnet ist. Die erste Küvettenwand 13 ist vorzugsweise optisch für eine vom Sensor gemessene Wellenlänge transparent, die zweite Küvettenwand 14 kann für eine von einem Strahler des Sensors erzeugte Wellenlänge, oder für die vom Sensor gemessene Wellenlänge optisch transparent sein, oder ein Spiegel zur Reflektion von Strahlung gegen die erste Küvettenwand 13 sein.

Besonders bevorzugt ist der Sensor 22 mit einer Steuerungseinheit 21 verbunden, die eingerichtet ist, zur Erzeugung von Steuersignalen in Reaktion auf Messwerte des Detektors für die Steuerung des Lasers 8, die Stellung des Spiegels 9, und/oder die Einstellung eines Dosierventils 15, das der Dosierung einer ersten und/oder zweiten Vorläuferverbindung dient, und zur Übermittlung der Steuersignale mit dem Laser 8, der Stelleinrichtung des Spiegels 9 und/oder Dosierventilen 15 mittels einer Datenleitung verbunden ist.

In einer weiter bevorzugten Ausführungsform ist die Auslassöffnung 3 über eine Rückführleitung 23, die vorzugsweise eine gesteuerte Pumpe enthält, mit der Einlassöffnung 2 verbunden, durch welche zumindest ein Teil des Trägerfluids gesteuert von der Auslassöffnung 3 zu Einlassöffnung 2 zurückgeführt wird, wenn das erfindungsgemäße Verfahren durchgerührt wird. In dieser Ausführungsform kann Trägerfluid durch den Abschnitt des Behälters 1 rezykliert werden, in welchem die Laserstrahlung auf den Metallkörper 6 gerichtet ist und einen Volumenabschnitt des Behälters 1 durchquert, wobei die Rückführung zumindest eines Teils des Trägerfluids zur Einwirkung des Laserstrahls auf bereits erzeugte Nanopartikel führt. In dieser Ausführungsform ist es bevorzugt, in einem ersten Verfahrensschritt Trägerfluid ohne Vorläuferverbindung durch den Strömungskanal hindurch treten zu lassen und den Metallkörper 6 mit Laserstrahlung zu bestrahlen, sodass die Rückführung von Trägerfluid von der Auslassöffnung 3 zur Einlassöffnung 2 zu einem Durchtritt von metallischen bzw. metalloxidischen Nanopartikeln, die im Trägerfluid suspendiert sind, durch den Volumenabschnitt des Behälters 1 führt, in welchem die Laserstrahlung durch den Behälter 1 tritt. Es hat sich gezeigt, dass eine Einwirkung von Laserstrahlung auf bereits erzeugte, im Trägerfluid suspendierte Nanopartikel zu einer gezielten Veränderung der Nanopartikel führt, insbesondere zu einer Reduzierung von deren Größe oder deren Größenverteilung und damit z.B. zu kleineren Partikeln, die bevorzugt eine homogene oder enge Größenverteilung aufweisen. Es ist dabei bevorzugt, in einem zweiten Verfahrensschritt erste 10 und/oder zweite Vorläufersubstanz durch eine zweite Einlassöffnung 16 in den als Strömungskanal ausgebildeten Behälter 1 einzubringen, wobei die Rückführung von Trägerfluid von Auslassöffnung 3 zur Einlassöffnung 2 gestoppt ist, und Trägerfluid durch die Auslassöffnung 3 austreten gelassen wird, vorzugsweise mit anschließender Abtrennung zumindest eines Teils des Trägerfluids von den erzeugten Konjugaten.

Figur 3 zeigt eine weitere bevorzugte Ausführungsform der erfindungsgemäßen Vorrichtung zur Verwendung im Herstellungsverfahren von Konjugaten, bei der innerhalb eines Gehäuses 20 ein als Strömungskanal ausgebildeter Behälter 1 angeordnet ist. Der Laser 8 erzeugt einen kontinuierlichen Laserstrahl oder einen gepulsten Laserstrahl, der mittels des steuerbaren Spiegels 9 durch einen transparenten Abschnitt der Wandung des Behälters 1 auf den Metallkörper 6 gerichtet ist. Der Spiegel 9 ist z.B. an einer von einer Steuereinheit gesteuerten Stelleinrichtung angebracht und bildet z.B. eine Abtasteinrichtung (Scanner). Eine erste Einlassöffnung 2 des als Strömungskanal ausgebildeten Behälters 1 ist mit einem Vorratsbehälter (nicht dargestellt) für Trägerfluid verbunden und wird mittels Druckbeaufschlagung, z.B. mittels einer Pumpe 5 erzeugt, mit Trägerfluid angeströmt. Das Trägerfluid kann mit Vorläuferverbindung versetzt sein, oder Vorläuferverbindung kann über zweite Einlassöffnungen 16 und/oder dritte Einlassöffnungen 18 zugeführt werden, insbesondere, wenn das Trägerfluid bei Einströmen in die erste Einlassöffnung 2 keine Vorläuferverbindung aufweist.

Die Leitungen, die die erste Einlassöffnung 2 mit einem ersten Vorratsbehälter (nicht gezeigt) für Trägerfluid, zweite Einlassöffnungen 16 und dritte Einlassöffnungen 18 mit dem jeweils zugeordneten zweiten bzw. dritten Vorratsbehälter (nicht gezeigt) verbinden, weisen vorzugsweise jeweils steuerbare Dosierventile 15 auf, die besonders bevorzugt mit einer Steuereinheit 21 verbunden sind, wie mit Bezug auf die Figur 2 beschrieben ist.

Der Strömungskanal weist in einem Wandungsabschnitt eine Fixiereinrichtung 7 auf, auf der ein Metallkörper 6 angeordnet ist. Der Wandungsabschnitt 11 gegenüber der Fixiereinrichtung 7 ist transparent für die Laserstrahlung.

Wie schematisch dargestellt, kann der Behälter 1 von einem Gehäuse 20 gebildet werden, das zumindest in dem Abschnitt teilbar ist, in dem die Fixiereinrichtung 7 angeordnet ist, um z.B. den Metallkörper 6 auf der Fixiereinrichtung zu positionieren.

### Beispiel 1: Herstellung eines Detektionskonjugats mit Nanogoldpartikel

Zur Herstellung eines Detektionskonjugats mit einem metallischen Nanopartikel wurde Goldfolie als Metallkörper in einer Vorrichtung nach Figur 1 in eine wässrige Lösung als Trägerflüssigkeit eingebracht, die eine Nukleinsäuresequenz entsprechend Seq.-ID Nr. 3 als Vorläuferverbindung enthielt. Die Goldfolie wurde mit 120 fs Laserimpulsen bei einer Wellenlänge von 800 nm bei einer maximalen Energie von 400 µJ pro Puls, Strahldurchmesser 4 mm bei einem Abstand von etwa 40 mm von der Linse zur Goldfolie bei einer Wiederholungsrate von 5 kHz bestrahlt. Der Energieeintrag auf die Goldfolie war ca. 100 µJ. Die wässrige Lösung enthielt ca. 3µM Nukleinsäuresequenz in Wasser, mit einer Schichthöhe von ca. 1 cm oberhalb der Goldfolie.

Die Analyse der Reaktionsprodukte durch Polyacrylamidgelektrophorese zeigt eine nur geringe Degradation der Nukleinsäuresequenz. Die Analyse der Reaktionsprodukte durch Transmissions- Elektronenmikroskopie ergab, dass die Konjugate eine Größenverteilung mit einem Mittel von ca. 5,2 bis 5,5 nm aufwiesen. Die Konjugate waren nicht agglomeriert und zeigten eine etwa sphärische Gestalt bei den verwendeten Parametern wurden ca. 20 µg/min Goldpartikel erzeugt, die ohne weitere chemische Kopplungsreagenzien eine stabile Bindung mit der Nukleinsäuresequenz eingingen.

Bei Wiederholung dieses Verfahrens mit höherem Energieeintrag mittels der Laserstrahlung ohne oder alternativ mit Bewegung der wässrigen Lösung wurden dieselben Konjugate erzeugt, wobei deren Analyse bei Bewegung der wässrigen Lösung einen geringeren Anteil an Konjugaten mit degradierter Nukleinsäuresequenz zeigte. Hier zeigt sich, dass die erfindungsgemäße Bewegung des Trägerfluids einen hohen Energieeintrag mittels Laserstrahlung auf den Metallkörper erlaubt, ohne eine signifikante Degradation von Konjugaten zu bewirken.

Das Verfahren wurde auch in einer Vorrichtung mit Strömungskanal entsprechend Figur 3 durchgeführt, wobei die Trägerflüssigkeit nicht rezykliert wurde. Die zweiten und dritten Einlassöfinungen waren verschlossen, die Auslassöffnung war mit einem Schlauch mit einem Sammelbehälter verbunden. Das in die erste Einlassöffnung 2 gepumpte Trägerflüssigkeit enthielt ein Oligonukleotid nach Seq.-ID Nr. 3 als Vorläuferverbindung und wurde mit einem Volumenstrom von 1 mL/min gefördert. Der Volumenabschnitt des Strömungskanals zwischen Metallkörper (Goldfolie) und transparentem Wandungsabschnitt betrug ca. 2 mL. Als Laser wurde ein Ultrakurzpulslaser mit einer Strahlungsleistung von ca. 200µJ bis 300µJ eingesetzt.

Für die Aufreinigung wurden erzeugte Konjugate in Trägerflüssigkeit mittels Zentrifugation von Oligonukleotiden getrennt, die nicht mit Oligonukleotiden verbunden waren und von nicht mit organischem Bestandteil verbundenen Nanopartikeln getrennt.

### Beispiel 2: Verwendung des Herstellungsverfahrens für Konjugate zur Detektion Y-chromosomenhaltiger Spermien in Frischsamen und deren geschlechtsspezifische Sortierung

Frisch gewonnener Bullensamen wurde in üblicher Weise in Verdünner verdünnt und mit Detektionskonjugat, das nach einer Variante von Beispiel 1 hergestellt wurde, 30 bis 120 min, vorzugsweise bei einer Temperatur von 20 °C bis 40 °C inkubiert und anschließend in einem Durchflusszytometer gemäß US 5125759 oder der DE 10 2005 044 530 mit Licht der jeweiligen Anregungswellenlänge (520 nm) für die Goldnanopartikel bestrahlt. Die Emission wurde gemessen.

Für die mit Detektionskonjugat nach Beispiel 1 spezifisch markierten Y-chromosomenhaltigen Spermatozoen wurde ein Lumineszenzsignal gemessen, das gegenüber dem Signal, das für die X-chromosomhaltigen Spermatozoen gemessen wurde, ein verschobenes Maximum aufwies. Dies zeigt, dass dieses Detektionskonjugat bei Hybridisierung der Nukleinsäuresequenz ein für den Analyten spezifisches Signal unter Bestrahlung bei Anregungswellenlänge erzeugt, während Zellen, die keine mit der Nukleinsäuresequenz des Detektionskonjugats hybridisierende Sequenz enthalten, ein abweichendes Signal unter Bestrahlung emittieren.

Für die mit Detektionskonjugat nach Beispiel 1 chromosomenspezifisch angefärbten Spermatozoen wurde eine Veränderung der detektierten Oberflächenplasmonresonanz für die Y-chromosomhaltigen Spermatozoen festgestellt, während die X-chromosomhaltigen Spermatozoen eine signifikant weniger veränderte Oberflächenplasmonresonanz zeigten.

### Bezugszeichenliste:

- 1: Behälter
- 2: erste Einlassöffnung
- 3: Auslassöffnung
- 4: Trägerfluid
- 5: Pumpeinrichtung
- 6: Metallkörper
- 7: Fixiereinrichtung
- 8: Laser
- 9: Spiegel
- 10: erste Vorläuferverbindung
- 11: transparenter Abschnitt
- 12: Küvettenabschnitt
- 13: erste Küvettenwand
- 14: zweite Küvettenwand
- 15: Dosierventil, Dosiereinrichtung
- 16: zweite Einlassöffnung
- 17: erster Vorratsbehälter
- 18: dritte Einlassöffnung
- 19: zweiter Vorratsbehälter
- 20: Gehäuse
- 21: Steuereinheit
- 22: Sensor
- 23: Rückführleitung

## Patentansprüche

1. Verfahren zur Herstellung von Konjugaten, die einen metallhaltigen nanopartikulären Bestandteil und einen organischen Bestandteil aufweisen, durch Bereitstellen eines Trägerfluids (4) in einem Behälter (1),
Bereitstellen eines Metallkörpers (6), der Metall oder Metalloxid aufweist, in dem Trägerfluid (4),
Erzeugung metallhaltiger Nanopartikel durch Bestrahlung der Oberfläche des Metallkörpers (6) mit einem Laserstrahl,
wobei das Trägerfluid (4) mittels einer Pumpeinrichtung (5) über die Oberfläche des Metallkörpers (6) bewegt wird,
und das Trägerfluid (4) mit einer Vorläuferverbindung des organischen Bestandteils versetzt wird.

2. Verfahren nach Anspruch 1, **dadurch gekennzeichnet, dass** die Laserstrahlung eine kontinuierliche Laserstrahlung ist.

3. Verfahren nach Anspruch 2, **dadurch gekennzeichnet, dass** die Laserstrahlung von einem CW-Laser (8) erzeugt wird.

4. Verfahren nach Anspruch 1, **dadurch gekennzeichnet, dass** die Laserstrahlung Ultrakurzpulslaserstrahlung ist.

5. Verfahren nach einem der voranstehenden Ansprüche, **dadurch gekennzeichnet, dass** das Trägerfluid (4) mit der Vorläuferverbindung versetzt wird, bevor es über die Oberfläche des Metallkörpers (δ) bewegt wird.

6. Verfahren nach einem der voranstehenden Ansprüche, **dadurch gekennzeichnet, dass** der Metallkörper (6) einstückiges oder partikuläres Metalloder Metalloxid ist.

7. Verfahren nach einem der voranstehenden Ansprüche, **dadurch gekennzeichnet, dass** das Trägerfluid (4) mit Vorläuferverbindung des organischen Bestandteils versetzt wird, nachdem es über die Oberfläche des Metallkörpers (6) bewegt wurde.

8. Verfahren nach einem der voranstehenden Ansprüche, **dadurch gekennzeichnet, dass** der Behälter (1) als Strömungskanal ausgebildet ist und der Metallkörper (6) in einem Abschnitt des Strömungskanals zwischen einer Einlassöffnung (2) an einem ersten Ende des Behälters (1) und einer Auslassöffnung (3) an einem zweiten Ende des Behälters (1) angeordnet ist, und das Trägerfluid (4) von der Einlassöffnung (2) zu der Auslassöffnung (3) bewegt wird.

9. Verfahren nach einem der voranstehenden Ansprüche, **dadurch gekennzeichnet, dass** das Trägerfluid (4) in turbulenter Strömung über die Oberfläche des Metallkörpers (6) bewegt wird.

10. Verfahren nach einem der voranstehenden Ansprüche, **dadurch gekennzeichnet, dass** dem Trägerfluid zumindest eine weitere Vorläuferverbindung eines organischen Bestandteils zugegeben wird, bevor oder nachdem das Trägerfluid (4) über die Oberfläche des Metallkörpers (6) bewegt wird.

11. Verfahren nach einem der voranstehenden Ansprüche, **dadurch gekennzeichnet, dass** das Trägerfluid (4) am Auslassende (3) gesammelt wird und nicht mit Nanopartikeln umgesetzte Vorläuferverbindung und/oder nicht mit Vorläuferverbindung umgesetzte Nanopartikel vom Konjugat abgetrennt wird.

12. Verfahren nach einem der Ansprüche 8 bis 11, **dadurch gekennzeichnet, dass** zumindest ein Teil des Trägerfluids vom Auslassende (3) zum Einlassende (2) rückgeführt wird.

13. Verfahren nach einem der voranstehenden Ansprüche, **gekennzeichnet durch** die Erzeugung eines Meßsignals bei Detektion wenigstens einer Eigenschaft des Trägerfluids (4) an einem Küvettenabschnitt (12) des Behälters (1).

14. Verfahren nach Anspruch 13, **dadurch gekennzeichnet, dass** die Detektion eine spektrometrische Detektion oder dynamische Lichstreuung ist

15. Verfahren nach Anspruch 12, **dadurch gekennzeichnet, dass** aus dem Meßsignal aus der Detektion des Trägerluids ein Steuersignal für die Laserbestrahlung erzeugt wird und dieses die Laserstrahlung und/oder eine Relativbewegung der Laserbestrahlung zum Metallkörper und/oder die Zugabe von Vorläuferverbindung in das Trägerfluid steuert.

16. Verfahren nach einem der voranstehenden Ansprüche, **dadurch gekennzeichnet, dass** die erste Vorläuferverbindung aus der Gruppe ausgewählt ist, die Oligonukleotide, Peptide, Polyether, Polyester, Polyamide und Monomeren mit zumindest einer reaktiven Gruppe umfasst, die ausgewählt ist unter ungesättigten C-C-Bindungen, Disulfid-, Thiol-, Keto-, Carboxy-, Phosphin-, Amin- und Amidgruppen.

17. Verfahren nach einem der voranstehenden Ansprüche, **dadurch gekennzeichnet, dass** das Metall Gold (Au) ist, und die erste Vorläuferverbindung ein geschlechtschromosomenspezifisches Oligonukleotid ist.

18. Verwendung eines Verfahrens nach einem der voranstehenden Ansprüche zur Identifikation eines intrazellulären oder extrazellulären Analyten, wobei die erste Vorläuferverbindung für den Analyten spezifisch ist und die Anwesenheit des metallhaltigen nanopartikulären Bestandteils des Konjugats an Zellen detektiert wird.

19. Verwendung nach Anspruch 18, **dadurch gekennzeichnet, dass** Zellen in Abhängigkeit von der Detektion des metallhaltigen nanopartikulären Bestandteils des Konjugats an Zellen durchflußzytometrisch in Fraktionen sortiert werden.

20. Vorrichtung zur Verwendung in einem Verfahren nach einem der Ansprüche 1 bis 15 mit einem Behälter (1) zur Aufnahme eines Trägerfluids (4),
einer mit dem Behälter (1) verbundenen Zirkulationseinrichtung zum Bewegen des Trägerfluids in dem Behälter (1) und einer innerhalb des Behälters (1) angeordneten Fixiereinrichtung (7) zur Fixierung eines Metallkörpers (6),
wobei der Behälter eine Einlassöffnung (2) zur Zuführung von Trägerfluid (4) und eine Auslassöffnung (3) zum Abführen von Trägerfluid (4) aufweist,
mit einem Laser (8), der eingerichtet ist, Laserstrahlung zu erzeugen und auf den Abschnitt der Fixiereinrichtung (7) zu richten, in welchem der Metallkörper (6) anzuordnen ist.

21. Vorrichtung nach Anspruch 20, **dadurch gekennzeichnet, dass** die Zirkulationseinrichtung eine am Behälter (1) angeschlossene Zirkulationsleitung mit einer Pumpeinrichtung ist, oder eine im Behälter (1) angeordnete Pumpeinrichtung (5).

22. Vorrichtung nach Anspruch 20 oder 21, **dadurch gekennzeichnet, dass** der Behälter (1) als Strömungskanal ausgebildet ist, der an seinem ersten Ende die Einlassöffnung (2) und an seinem gegenüberliegenden zweiten Ende die Auslassöffnung (3) aufweist, wobei die Fixiereinrichtung (7) in einem Abschnitt des Strömungskanals zwischen der Einlassöffnung (2) und Auslassöffnung (3) angeordnet ist, und der Strömungskanal einen für die vom Laser (8) erzeugte Laserstrahlung transparenten Abschnitt (11) aufweist, durch welchen die Laserstrahlung gerichtet ist.

23. Vorrichtung nach einem der Ansprüche 20 bis 22, **dadurch gekennzeichnet, dass** der Strömungskanal einen Küvettenabschnitt (12) aufweist, der eine erste Küvettenwand (13) und eine beabstandete zweite Küvettenwand (14) aufweist, und an der ersten Küvettenwand (13) ein Sensor (22) zur Messung wenigstens einer Eigenschaft des Trägerfluids (4) und/oder eines Konjugats angeordnet ist.

24. Vorrichtung nach Anspruch 23, **dadurch gekennzeichnet, dass** die erste Küvettenwand (13) optisch transparent ist und der Sensor (22) ein Photometer ist.

25. Vorrichtung nach Anspruch 23 oder 24, **dadurch gekennzeichnet, dass** die zweite Küvettenwand (14) optisch transparent ist und eine Strahlungsquelle an der zweiten Küvettenwand (14) angeordnet und gegen die erste Küvettenwand (13) gerichtet ist.

26. Vorrichtung nach Anspruch 23 oder 24, **dadurch gekennzeichnet, dass** die zweite Küvettenwand (14) ein Spiegel ist.

27. Vorrichtung nach einem der Ansprüche 18 bis 23, **dadurch gekennzeichnet, dass** an die Auslassöffnung (3) eine mit einem Ventil versehene Rückführleitung (23) angeschlossen ist, die mit der Einlassöffnung (2) verbunden ist.

28. Vorrichtung nach einem der Ansprüche 20 bis 27, **dadurch gekennzeichnet, dass** der Laser (8) eingerichtet ist, eine kontinuierliche Laserstrahlung zu erzeugen.

29. Vorrichtung nach einem Ansprüche 20 bis 28, **dadurch gekennzeichnet, dass** der Behälter (1) in einem Abschnitt zwischen Fixiereinrichtung (7) und Austassöffnung (3) eine zweite Einlassöffnung (16) aufweist, die mit zwischengeschaftetem Dosiereinrichtung (15) mit einem ersten Vorratsbehälter (17) zur Aufnahme einer ersten und/oder zweiten Vorläuferverbindung verbunden ist, und/oder eine dritte Einlassöffnung (18) in einem Abschnitt zwischen der Einlassöffnung (2) und der Fixiereinrichtung (7) aufweist, die mit zwischengeschaltetem Dosiereinrichtung (15) mit einem zweiten Vorratsbehälter (19) zur Aufnahme einer ersten oder zweiten Vorläuferverbindung verbunden ist.

30. Vorrichtung nach einem der Ansprüche 20 bis 29, **dadurch gekennzeichnet, dass** der Sensor (22) zur Übertragung von Meßsignalen mit einer Steuerungseinheit (21) verbunden ist, die eingerichtet ist, vom Sensor (22) empfangene Signale zu verarbeiten und Steuerungssignale zur Steuerung von Dosiereinrichtungen (15), Laser (8) und/oder Stellung des Spiegels (9) und/oder eines Ventils (15) in der Rückfuhrleitung (23) zu erzeugen und mit diesen für die Übermittlung von Steuersignalen verbunden ist.

## Claims

1. Process for the production of conjugates having a metal containing nanoparticular component and an organic component by providing a carrier fluid (4) in a receptacle (1),
providing a metal body (6) comprising metal or metal oxide within the carrier fluid (4),
generation of metal containing nanoparticles by irradiation of the surface of the metal body (6) with a laser beam,
wherein the carrier fluid (4) is moved over the surface of the metal body (6) by means of a pumping device (5),
and a precursor compound of the organic component is added to the carrier fluid (4).

2. Process according to claim 1, **characterized in that** the laser radiation is a continuous laser radiation.

3. Process according to claim 2, **characterized in that** the laser radiation is generated by a CW laser (8).

4. Process according to claim 1, **characterized in that** the laser radiation is ultrashort pulse laser radiation.

5. Process according to one of the preceding claims, **characterized in that** the precursor compound is added to the carrier fluid (4) before it is moved over the surface of the metal body (6).

6. Process according to one of the preceding claims, **characterized in that** the metal body (6) is single-piece or particular metal or metal oxide.

7. Process according to one of the preceding claims, **characterized in that** precursor compound of the organic component is added to the carrier fluid (4) after it was moved over the surface of the metal body (6).

8. Process according to one of the preceding claims, **characterized in that** the receptacle (1) is formed as a flow channel and the metal body (6) is arranged in a section of the flow channel between an inlet (2) at a first end of the receptacle (1) and an outlet (3) at a second end of the receptacle (1), and the carrier fluid (4) is moved from the inlet (2) to the outlet (3).

9. Process according to one of the preceding claims, **characterized in that** the carrier fluid (4) is moved in a turbulent flow over the surface of the metal body (6).

10. Process according to one of the preceding claims, **characterized in that** at least one further precursor compound of an organic component is added to the carrier fluid before or after the carrier fluid (4) is moved over the surface of the metal body (6).

11. Process according to one of the preceding claims, **characterized in that** the carrier fluid (4) is collected at the outlet end (3) and that precursor compound not converted with nanoparticles and/or nanoparticles not converted with precursor compound are separated from the conjugate.

12. Process according to one of the claims 8 to 11, **characterized in that** at least portion of the carrier fluid is returned from the outlet end (3) to the inlet end (2).

13. Process according to one of the preceding claims, **characterized by** the generation of a measuring signal upon detection of at least one property of the carrier fluid (4) at a cuvette section (12) of the receptacle (1).

14. Process according to claim 13, **characterized in that** the detection is a spectrometric detection or dynamic light scatter.

15. Process according to claim 12, **characterized in that** a control signal for the laser irradiation is generated from the measuring signal from the detection of the carrier fluid and that this control signal controls the laser radiation and/or a relative motion of the laser irradiation to the metal body and/or the addition of precursor compound into the carrier fluid.

16. Process according to one of the preceding claims, **characterized in that** the first precursor compound is selected from the group comprising oligonucleotides, peptides, polyethers, polyesters, polyamides and monomers having at least one reactive group selected from unsaturated C-C-bonds, bisulfide, thiol, keto, carboxyl, phosphine, amine and amide groups.

17. Process according to one of the preceding claims, **characterized in that** the metal is gold (Au) and the first precursor compound is a sex chromosome-specific oligonucleotide.

18. Use of a process according to one of the preceding claims for identification of an intracellular or extracellular analyte, wherein the first precursor compound is specific for the analyte and the presence of the metal containing nanoparticular component of the conjugate at cells is detected.

19. Use according to claim 18, **characterized in that** cells are sorted flow-cytometrically into fractions in dependence from the detection of the metal containing nanoparticular component of the conjugate at cells.

20. Device for use in a process according to one of the claims 1 to 15 with a receptacle (1) for receiving a carrier fluid (4),
a circulation device connected to the receptacle (1) for movement of the carrier fluid in the receptacle (1) and a fixation device (7) arranged within the receptacle (1) for fixation of a metal body (6),
wherein the receptacle has an inlet (2) for addition of carrier fluid (4) and an outlet (3) for removal of carrier fluid (4),
with a laser (8) which is disposed to generate and direct laser radiation onto the section of the fixation device (7) in which the metal body (6) is to be arranged.

21. Device according to claim 20, **characterized in that** the circulation device is a circulation duct connected to the receptacle (1) and having a pumping device, or a pumping device (5) arranged within the receptacle (1).

22. Device according to claim 20 or 21, **characterized in that** the receptacle (1) is formed as a flow channel which at its first end has the inlet opening (2) and has an outlet opening (3) at its opposite second end, wherein the fixation device (7) is arranged in a section of the flow channel between the inlet opening (2) and outlet opening (3), and the flow channel has a section (11) transparent for the laser radiation generated by the laser (8), through which section the laser radiation is directed.

23. Device according to one of the claims 20 to 22, **characterized in that** the flow channel has a cuvette section (12) having a first cuvette wall (13) and a spaced second cuvette wall (14), and a sensor (22) for measurement of at least one property of the carrier fluid (4) and/or of a conjugate is arranged at the first cuvette wall (13).

24. Device according to claim 23, **characterized in that** the first cuvette wall (13) is optically transparent and the sensor (22) is a photometer.

25. Device according to claim 23 or 24, **characterized in that** the second cuvette wall (14) is optically transparent and a radiation source is arranged at the second cuvette wall (14) and is directed against the first cuvette wall (13).

26. Device according to claim 23 or 24, **characterized in that** the second cuvette wall (14) is a mirror.

27. Device according to one of the claims 18 to 23, **characterized in that** at the outlet opening (3) a return duct (23) provided with a valve is connected which is connected to the inlet opening (2).

28. Device according to one of the claims 20 to 27, **characterized in that** the laser (8) is disposed for generating a continuous laser radiation.

29. Device according to one of the claims 20 to 28, **characterized in that** the receptacle (1) in a section between fixation device (7) and outlet opening (3) has a second inlet opening (16) which by means of an interconnected dosing device (15) is connected to a first storage receptacle (17) for receiving a first and/or a second precursor compound, and/or in a section between the inlet opening (2) and the fixation device (7) has a third inlet opening (18) which by means of an interconnected dosing device (15) is connected to a second storage receptacle (19) for receiving a first or second precursor compound.

30. Device according to one of the claims 20 to 29, **characterized in that** for transmission of measuring signals the sensor (22) is connected to a control unit (21) which is disposed for processing signals received by the sensor (22) and for generating control signals for controlling of dosing devices (15), laser (8) and/or the position of the mirror (9) and/or a valve (15) in the return duct (23) and which is connected with these for the transmission of control signals.

## Revendications

1. Procédé pour la réalisation de produits de conjugaison, qui comportent un constituant nanoparticulaire contenant du métal et un constituant organique, moyennant la mise à disposition d'un fluide porteur (4) dans un récipient (1),
la mise à disposition d'un corps métallique (6), comportant du métal ou un oxyde métallique, dans le fluide porteur (4),
la production de nanoparticules contenant du métal par irradiation de la surface du corps métallique (6) au moyen d'un faisceau laser,
le fluide porteur (4) étant déplacé au-dessus de la surface du corps métallique (6) au moyen d'un dispositif de pompage (5),
et le fluide porteur (4) étant mélangé à un composé précurseur du constituant organique.

2. Procédé selon la revendication 1, **caractérisé en ce que** le faisceau laser est un faisceau laser continu.

3. Procédé selon la revendication 2, **caractérisé en ce que** le faisceau laser est généré par un laser continu (8).

4. Procédé selon la revendication 1, **caractérisé en ce que** le faisceau laser est un faisceau laser à impulsions ultracourtes.

5. Procédé selon l'une quelconque des revendications précédentes, **caractérisé en ce que** le fluide porteur (4) est mélangé au composé précurseur avant qu'il soit déplacé au-dessus de la surface du corps métallique (6).

6. Procédé selon l'une quelconque des revendications précédentes, **caractérisé en ce que** le corps métallique (6) est un métal ou un oxyde métallique en une seule pièce ou sous forme particulaire.

7. Procédé selon l'une quelconque des revendications précédentes, **caractérisé en ce que** le fluide porteur (4) est mélangé au composé précurseur du constituant organique après qu'il a été déplacé au-dessus de la surface du corps métallique (6).

8. Procédé selon l'une quelconque des revendications précédentes, **caractérisé en ce que** le récipient (1) est réalisé sous forme de conduit d'écoulement et le corps métallique (6) est disposé dans un tronçon du conduit d'écoulement entre une ouverture d'admission (2) sur une première extrémité du récipient (1) et une ouverture d'évacuation (3) sur une deuxième extrémité du récipient (1), et le fluide porteur (4) est déplacé depuis l'ouverture d'admission (2) vers l'ouverture d'évacuation (3).

9. Procédé selon l'une quelconque des revendications précédentes, **caractérisé en ce que** le fluide porteur (4) est déplacé sous forme d'écoulement turbulent au-dessus de la surface du corps métallique (6).

10. Procédé selon l'une quelconque des revendications précédentes, **caractérisé en ce qu'**au moins un autre composé précurseur d'un constituant organique est ajouté au fluide porteur (4) avant ou après que le fluide porteur (4) ait été déplacé au-dessus de la surface du corps métallique (6).

11. Procédé selon l'une quelconque des revendications précédentes, **caractérisé en ce que** le fluide porteur (4) est collecté au niveau de l'ouverture d'évacuation (3) et est séparé du composé précurseur non transformé avec des nanoparticules et/ou des nanoparticules du produit de conjugaison non transformées avec le composé précurseur.

12. Procédé selon l'une quelconque des revendications 8 à 11, **caractérisé en ce qu'**au moins une partie du fluide porteur est renvoyée depuis l'ouverture d'évacuation (3) vers l'ouverture d'admission (2).

13. Procédé selon l'une quelconque des revendications précédentes, **caractérisé par** la production d'un signal de mesure en cas de détection d'au moins une propriété du fluide porteur (4) au niveau d'une partie de cuvette (12) du récipient (1).

14. Procédé selon la revendication 13, **caractérisé en ce que** la détection est une détection spectrométrique ou une diffusion dynamique de la lumière.

15. Procédé selon la revendication 12, **caractérisé en ce qu'**un signal de commande pour le faisceau laser est généré à partir du signal de mesure résultant de la détection du fluide porteur, et est destiné à commander le faisceau laser et/ou un mouvement relatif du faisceau laser par rapport au corps métallique et/ou l'addition du composé précurseur dans le fluide porteur.

16. Procédé selon l'une quelconque des revendications précédentes, **caractérisé en ce que** le premier composé précurseur est choisi dans le groupe comprenant des oligonucléotides, des peptides, des polyéthers, des polyesters, des polyamides et des monomères avec au moins un groupe réactif choisi parmi les liaisons C-C insaturées, les groupes disulfides, les groupes thiol, les groupes céto, les groupes carboxyles, les groupes phosphines, les groupes amines et les groupes amides.

17. Procédé selon l'une quelconque des revendications précédentes, **caractérisé en ce que** le métal est de l'or (Au) et le premier composé précurseur est un oligonucléotide spécifique du gonosome.

18. Utilisation d'un procédé selon l'une quelconque des revendications précédentes pour l'identification d'un analyte intracellulaire ou extracellulaire, le premier composé précurseur est spécifique pour l'analyte et la présence du constituant nanoparticulaire à teneur en métal du produit de conjugaison est détectée sur des cellules.

19. Utilisation selon la revendication 18, **caractérisée en ce que** des cellules sont triées en fractions par la cytométrie en flux en fonction de la détection du constituant nanoparticulaire à teneur en métal du produit de conjugaison sur des cellules.

20. Dispositif à utiliser dans un procédé selon l'une quelconque des revendications 1 à 15, comportant un récipient (1) pour recevoir un fluide porteur (4), un dispositif de circulation relié au récipient (1) et permettant de mettre en mouvement le fluide porteur dans le récipient (1), et un dispositif de fixation (7) disposé à l'intérieur du récipient (1) pour fixer un corps métallique (6), ledit récipient comportant une ouverture d'admission (2) pour l'admission du fluide porteur (4) et une ouverture d'évacuation (3) pour l'évacuation du fluide porteur (4), comportant un laser (8) qui est configuré pour émettre un faisceau laser et le diriger vers la partie du dispositif de fixation (7), dans laquelle doit être placé le corps métallique (6).

21. Dispositif selon la revendication 20, **caractérisé en ce que** le dispositif de circulation est une conduite de circulation raccordée au récipient (1) et munie d'un dispositif de pompage, ou un dispositif de pompage (5) raccordé au récipient (1).

22. Dispositif selon la revendication 20 ou 21, **caractérisé en ce que** le récipient (1) est réalisé sous la forme d'un conduit d'écoulement comportant une ouverture d'admission (2) au niveau de sa première extrémité et une ouverture d'évacuation (3) au niveau de sa deuxième extrémité située en face, le dispositif de fixation (7) étant disposé dans un tronçon du conduit d'écoulement entre l'ouverture d'admission (2) et l'ouverture d'évacuation (3), et le conduit d'écoulement comporte un tronçon (11), qui est transparent pour le faisceau laser généré par le laser (8) et à travers lequel est dirigé le faisceau laser.

23. Dispositif selon l'une quelconque des revendications 20 à 22, **caractérisé en ce que** le conduit d'écoulement comporte une partie de cuvette (12), comprenant une première paroi (13) et une deuxième paroi (14), écartée de celle-ci, et sur la première paroi (13) est agencé un capteur (22) pour mesurer au moins une propriété du fluide porteur (4) et/ou d'un produit de conjugaison.

24. Dispositif selon la revendication 23, **caractérisé en ce que** la première paroi (13) de la cuvette est optiquement transparente et le capteur (22) est un photomètre.

25. Dispositif selon la revendication 23 ou 24, **caractérisé en ce que** la deuxième paroi (14) de la cuvette est optiquement transparente, et une deuxième source de rayonnement est disposée sur la deuxième paroi (14) et est dirigée vers la première paroi (13).

26. Dispositif selon la revendication 23 ou 24, **caractérisé en ce que** la deuxième paroi (14) de la cuvette est un miroir.

27. Dispositif selon l'une quelconque des revendications 18 à 23, **caractérisé en ce que** l'ouverture d'évacuation (3) est raccordée à une conduite de retour (23), munie d'une vanne et reliée à l'ouverture d'admission (2).

28. Dispositif selon l'une quelconque des revendications 20 à 27, **caractérisé en ce que** le laser (8) est configuré pour générer un faisceau laser continu.

29. Dispositif selon l'une quelconque des revendications 20 à 28, **caractérisé en ce que** le récipient (1) comporte, dans un tronçon entre le dispositif de fixation (7) et l'ouverture d'évacuation (3), une deuxième ouverture d'admission (16) qui est reliée à un dispositif de dosage (15) intercalé, muni d'un premier réservoir de stockage (17) pour recevoir un premier et/ou un deuxième composé précurseur, et/ou comporte, dans un tronçon entre l'ouverture d'admission (2) et le dispositif de fixation (7), une troisième ouverture d'admission (18) qui est reliée à un dispositif de dosage (15) intercalé, muni d'un deuxième réservoir de stockage (19) pour recevoir un premier et/ou un deuxième composé précurseur.

30. Dispositif selon l'une quelconque des revendications 20 à 29, **caractérisé en ce que** le capteur (22), pour transmettre des signaux de mesure, est relié à une unité de commande (21) qui est configurée pour traiter des signaux reçus du capteur (22) et pour générer des signaux de commande pour commander les dispositifs de dosage (15), le laser (8) et/ou la position du miroir (9) et/ou une vanne (15) dans la conduite de retour (23), et qui est reliée à ceux-ci pour la transmission des signaux de commande.
